(19) Europäisches Patentamt
European Patent Office
Office européen des brevets

(11) EP 2 265 725 B1

(12) **EUROPEAN PATENT SPECIFICATION**

(45) Date of publication and mention of the grant of the patent:
**30.07.2014 Bulletin 2014/31**

(21) Application number: **08753189.3**

(22) Date of filing: **25.04.2008**

(51) Int Cl.:
***C12P 17/06*** *(2006.01)* ***C12N 1/20*** *(2006.01)*
***A61K 31/35*** *(2006.01)*

(86) International application number:
**PCT/KR2008/002375**

(87) International publication number:
**WO 2009/131264 (29.10.2009 Gazette 2009/44)**

(54) **Method for preparing 7,5,3',4'-tetrahydroxyisoflavone using a biotransformation system**

Verfahren zur Herstellung von 7,5,3',4'-Tetrahydroxyisoflavon unter Verwendung eines Biotransformationssystems

Procédé de préparation 7,5,3',4'-tetrahydroxyisoflavone au moyen d'un système de biotransformation

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**29.12.2010 Bulletin 2010/52**

(73) Proprietor: **Amorepacific Corporation**
**Seoul 140-777 (KR)**

(72) Inventors:
- **PARK, Jun Seong**
**Suwon-si**
**Gyeonggi-do 443-470 (KR)**
- **PARK, Hye Yoon**
**Anyang-si**
**Gyeonggi-do 431-713 (KR)**
- **RHO, Ho Sik**
**Yongin-si**
**Gyeonggi-do 446-903 (KR)**
- **KIM, Duck Hee**
**Seoul 137-030 (KR)**
- **CHANG, Ih Seop**
**Yongin-si**
**Gyeonggi-do 448-759 (KR)**
- **KIM, Byung Gee**
**Seoul 137-070 (KR)**
- **ROH, Chang hyun**
**Daejeon 302-803 (KR)**
- **SEO, Su Hyun**
**Seoul 151-817 (KR)**
- **CHOI, Kwon Young**
**Seoul 134-777 (KR)**
- **KIM, June Hyung**
**Seoul 151-050 (KR)**
- **CHA, Min-Ho**
**Seoul 151-812 (KR)**
- **BISHNU, Prasad Pandey**
**Rupandehi (NP)**

(74) Representative: **Ilgart, Jean-Christophe**
**BREVALEX**
**95 rue d'Amsterdam**
**75378 Paris Cedex 8 (FR)**

(56) References cited:
**US-A- 3 086 912 US-A- 4 537 859**
**US-A- 5 292 647**

- **B. Pandey et al: "Bioconversion of daidzein to ortho dihydroxy isoflavone by recombinant Streptomyces avermitilis", , 2007, XP002696008, Retrieved from the Internet: URL:http: //www.earticle.net/article.aspx?s n=100153 [retrieved on 2013-04-24]**
- **C. Roh et al: Isolation of novel hydroxylated isoflavones, ODI (ortho-dihydroxyisoflavone) from traditional Korean soybean paste (Doenjang) and isoflavone's biotransformation study , 2007, XP002696009, Retrieved from the Internet: URL:http://www.earticle.net/ article.aspx?s n=98606 [retrieved on 2013-04-24]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 265 725 B1

- KANKI KOMIYAMA ET AL: "Isolation of isoflavonoids possessing antioxidant activity from the fermentation broth of Streptomyces sp.", THE JOURNAL OF ANTIBIOTICS, vol. 42, no. 9, 1989, pages 1344-1349, XP055061086, ISSN: 0021-8820, DOI: 10.7164/antibiotics.42.1344
- SHINJI FUNAYAMA ET AL: "Structural study of isoflavonoids possessing antioxidant activity isolated from the fermentation broth of Streptomyces sp.", THE JOURNAL OF ANTIBIOTICS, vol. 42, no. 9, 1989, pages 1350-1355, XP055061079, ISSN: 0021-8820, DOI: 10.7164/antibiotics.42.1350
- R KING ET AL.: "Plasma and urinary kinetics of the isoflavones daidzein and genistein after a single soy meal in humans", AM J CLIN NUTR, vol. 67, 1998, pages 867-872, XP002696104,
- 'Spring symposium of the Korean Society of Industrial and Engineering Chemistry, May 11-12, 2007', article KIM, J.H.: 'ODI(o-dihydroxyisoflavone) biosysthesis and ODI production from Doenjang (Fermented Sybean paste) and Streptomyces avermitilis'
- MAATOOQ, G.T. ET AL.: 'Metabolism of daidzein by Nocardia species NRRL 5646 and Mortierella isabellina ATCC 38063' PHYTOCHEMISTRY vol. 66, 2005, pages 1007 - 1011, XP004894488
- SEEGER, M. ET AL.: 'Biotransformation of Natural and Synthetic Isoflavonoids by Two Recombinant Microbial Enzymes' AMERICAN SOCIETY FOR MICROBIOLOGY vol. 69, no. 9, September 2003, pages 5045 - 5050, XP002980920
- PARK, J-S. ET AL.: 'ortho-Dihydroxyisoflavone derivatives from aged Doenjan (Korean fermented soypaste) and its radical scavenging activity' BIOORGANIC & MEDICINAL CHEMISTRY LETTERS vol. 18, 2008, pages 5006 - 5009, XP025407659

## Description

[Technical Field]

The present invention relates to a method for preparing 7,5,3',4'-tetrahydroxyisoflavone using a biotransformation system, and more particularly to a method for preparing 7,5,3',4'-tetrahydroxyisoflavone, which comprises biotransforming daidzein or genistein by the actinomycete microorganism *Streptomyces avermitilis,* in order to efficiently prepare 7,5,3',4'-tetrahydroxyisoflavone having an excellent antioxidant function and a whitening effect.

[Background Art]

Isoflavones which are vegetable compounds contained mainly in beans are present as glucosides containing isoflavones as aglycons and are transformed into aglycons by microbial metabolism during fermentation processes. Isoflavones are known to have anticancer, antioxidant, antiatherogenic, blood glucose-lowering and osteoporosis-preventing effects, and thus studies thereon are being actively conducted. Among isoflavones, particularly 7,8,4'-trihydroxyisoflavone, 7,6,4'-trihydroxyisoflavone, 7,3',4'-trihydroxyisoflavone and 7,5,3',4'-tetrahydroxyisoflavone, which are ortho-dihydroxyisoflavones (ODIs), are highly valuable, because they have high antioxidant effects compared to those of other isoflavones and are difficult to synthesize chemically.

Daidzein and genistein are diphenolic phytoestrogen compounds found in numerous plants and soybeans. Such compounds were reported to have antioxidant, antimicrobial and metal chelating effects (Middleton et al., 1992; Dixon et al., 2002). Furthermore, they are being used as medical or chemical therapeutic agents for human health (Foti et al., 2005). Recently, interest in position-specific hydroxylated compounds of daidzein or genistein has increased. Ortho-specific hydroxylated isoflavones were reported to have biological effects higher than those of daidzein or genistein (Rufer and Kulling, 2006) and are known to anti-inflammatory and anti-allergenic activities (Rufer and Kulling, 2006). Also, they are tyrosine kinase inhibitors, have anticarcinogenic properties (Akiyama et al., 1987) and are used as potent tyrosinase inhibitors and lipoxygenase inhibitors (Chang et al., 2005; Voss et al., 1992). In addition, the use thereof as compounds for reducing the cause of cancer-related diseases was reported (Klus and Barz, 1995; Coward et al., 1993). Such hydroxylated compounds are difficult to synthesize by organic chemical methods, and thus can be produced only by either extraction from natural materials or biosynthesis methods.

As described above, hydroxylated isoflavones can be isolated and purified only by either extraction from natural materials or biosynthesis using microorganisms. In the case of extraction from natural materials, daidzein and genistein are easily obtained as the main compounds of isoflavones, but the hydroxylated forms of daidzein and genistein are not so. In other words, ortho-dihydroxyisoflavones which are contained in soybeans or plants are obtained in low yield, and the biosynthesis thereof using microorganisms have also not been much studied.

B. Pandey et al: "Bioconversion of daidzein to ortho dihydroxy isoflavone by recombinant Streptomyces avermitilis", 2007,

URL:http://www.earticle.net/article.aspx?sn=1001531 discloses the preparation of 7,3',4'-trihydroxyisoflavone by recombinant Streptomyces avermitilis.

C. Roh et al: "Isolation of novel hydroxylated isoflavones, ODI (ortho-dihydroxyisoflavone) from traditional Korean soybean paste (Doenjang) and isoflavone's biotransformation study", 2007, URL:http://www.earticle.net/article.aspx?sn=98606 discloses the preparation of 7,8,4'-trihydroxyisoflavone (8-OHD), 7,6,4'-trihydroxyisoflavone (6-OHD), 7,3',4'-trihydroxyisoflavone (3'-OHD) with Streptomyces avermitilis MA4680.

K. Komiyama et al: "Isolation of isoflavonoids possessing antioxidant activity from the fermentation broth of Streptomyces sp.", The Journal of Antibiotics, vol. 42, no. 9, 1989, pages 1344-1349, ISSN: 0021-8820, DOI: 10.7164/antibiotics.42.1344 and S. Funayama et al.: "Structural study of isoflavonoids possessing antioxidant activity isolated from the fermentation broth of Streptomyces sp.", The Journal of Antibiotics, vol. 42, no. 9, 1989, pages 1350-1355, ISSN: 0021-8820, DOI: 10.7164/antibiotics.42.1350 disclose the preparation of 7,8,4'-trihydroxyisoflavone and 7,3',4'-trihydroxyisoflavone.

J.H. Kim: "ODI (o-dihydroxyisoflavone) biosynthesis and ODI production from Doenjang (Fermented Soybean paste) and Streptomyces avermitilis", "Spring symposium of the Korean Society of Industrial and Engineering Chemistry, May 11-12, 2007", discloses the preparation of 7,8,4'-trihydroxyisoflavone (8-OHD), 7,6,4'-trihydroxyisoflavone (6-OHD), 7,3',4'-trihydroxyisoflavone (3'-OHD) with a microorganism from the genus Streptomyces.

In the prior art, hydroxylated compounds extracted from natural materials in low yield, and hydroxylated compounds obtained using microorganisms having low reactivity were analyzed, but there was no report of the productivity of 7,5,3',4'-tetrahydroxyisoflavone by microorganisms. Also, the reactivity of ortho-dihydroxyisoflavones with animal liver cells was reported, but there was a problem of low productivity due to low reactivity (Kulling et al, 2001).

[Disclosure]

[Technical Problem]

Accordingly, the present inventors have found that 7,5,3',4'-tetrahydroxyisoflavone having an antioxidant function and a whitening effect can be efficiently prepared by biotransforming daidzein and genistein by the actinomycete microorganisms *Streptomyces avermitilis.*

It is, therefore, an object of the present invention to provide a method of preparing 7,5,3',4'-tetrahydroxyisoflavone by biotransforming daidzein and genistein.

[Technical Solution]

To achieve the above object, the present invention provides a method of preparing 7,5,3',4'-tetrahydroxyisoflavone by biotransforming daidzein and genistein by the actinomycete microorganisms *Streptomyces* avermitilis.

[Advantageous Effects]

According to the present invention, there can be provided a preparation method of specifically producing and accumulating ODIs, which are antioxidant substances or whitening substances, using actinomycete microorganisms, and furthermore, biosynthesizing modified compounds. The present invention will become a high value-added invention in scientific research and industrial application.

[Description of Drawings]

FIG. 1 shows the results of HPLC analysis of each test substance. (1): 7,3',4'-trihydroxyisoflavone as a metabolite; peak (2): daidzein as a substrate; peak (3): 7,5,3',4'-tetrahydroxyisoflavone as a metabolite; and peak (4): genistein as a substrate. The HPLC analysis was performed in the following conditions: UV: 254 nm; flow rate: 1 ml/min; and solvent used: ACN:DW=3:7 (containing 1% TFA).
FIG. 2 shows the results of NMR analysis of 7,3',4'-trihydroxyisoflavone.
FIG. 3 shows the results of NMR analysis of 7,5,3',4'-tetrahydroxyisoflavone.
FIG. 4 shows reactors for biotransforming daidzein using *Streptomyces avermitilis.* (A): a prior art reactor; and (B): a batch reactor used in the present invention to make oxygen supply smooth.
FIG. 5 shows the results of the GC-MS analysis of substrate daidzein and reaction products, carried out using BSTFA derivatization. 1): daidzein, room temperature, 18.5 min, MS 398; 2) 7,3',4'-trihydroxyisoflavone, room temperature, 24.4 min, MS 486; 3) 7,8,4'-trihydroxyisoflavone, room temperature, 25.1 min, MS 486; and 4) 7,6,4'-trihydroxyisoflavone, room temperature, 26.8 min, MS 486.
FIG. 6 shows the structures of various modified isoflavones produced by biotransforming daidzein by *Streptomyces avermitilis.*
FIG. 7 shows the results of the reaction of *Streptomyces avermitilis* with daidzein and the results of GC analysis (reaction conditions: use of coils, 220 rpm, reaction time: 1 month, and use of R2YE medium).
FIG. 8 is a schematic diagram showing a process of derivatization by BSTFA for analysis.
FIGS. 9 and 10 show the results of MS spectrum analysis of test substances.

[Best Mode]

Hereinafter, the present invention will be described in further detail.

The present invention relates to a method of preparing 7,5,3',4'-tetrahydroxyisoflavone using the actinomycete microorganisms streptomyces avermitilis. In the present invention, microbial strains capable of preparing ortho-dihydroxyisoflavones using daidzein and genistein as substrates were first screened.

Microbial strains which screened in the present invention include *Streptomyces avermitilis, Nocardia farcinica* and *Streptomyces lincolnesis.* Among these strains, *Streptomyces avermitilis* showed the highest ortho-dihydroxyisoflavone productivity and also showed the position-specific hydroxylation at the 3' position of genistein.

Although the terms used in the present invention are conventionally used in the art and the meaning thereof can be understood by any person skilled in the art, the terms are briefly defined as follows:

1) ODI: ortho-dihydroxyisoflavone.

2) ISP2 medium (per 1 liter): 5 g malt extract, 2 g yeast extract and 2 g glucose.

3) YEME medium (per 1 liter): 3 g malt extract, 3 g yeast extract, 5 g peptone, 300 g sucrose, 2 ml $MgCl_2 \cdot 6H_2O$ (2.5M) and 25 ml glycine (20%).

4) R2YE medium: 103 g sucrose, 10 g glucose, 0.25 g $K_2SO_4$, 5 g yeast extract, 0.1 g Difco casamino acid, 100 ml TES buffer (5.73%, pH 7.2), 10 ml $KH_2PO_4$ (0.5%), 80 ml $CaCl_2 \cdot 2H_2O$ (3.68%), 15 ml L-proline (20%), 2 ml trace element solution and 5 ml NaOH(1N).

5) HPLC: High-Performance Liquid Chromatography.

6) GC-MS: Gas Chromatography-Mass Spectrometry.

7) NMR: Nuclear Magnetic Resonance spectroscopy.

8) BSTFA: a derivative for GC analysis. N,O-bis(trimethylsilyl)trifluoroacetamide).

9) rpm: revolutions per minute or the revolutions per minute of a disc.

The method comprises a step of biotransforming daidzein and genistein by the screened strains. Namely, it comprises a biotransformation process of the following reaction scheme 1 of preparing 3'-specific hydroxylated compounds from daidzein and genistein using the screened strains:

[Reaction Scheme 1]

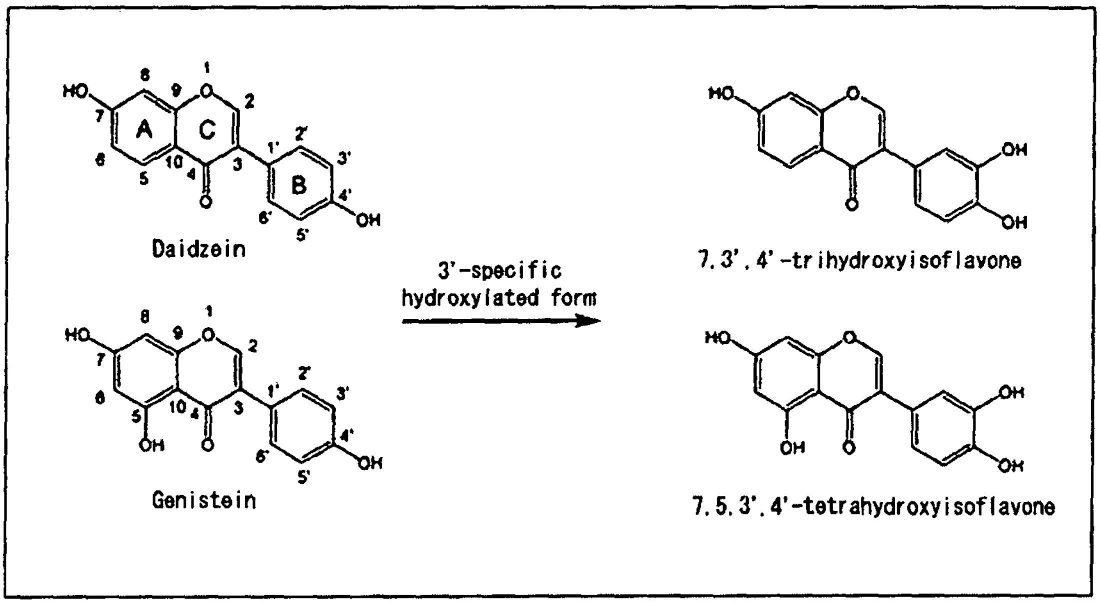


The following reaction scheme 2 shows a process of producing ortho-specific hydroxylated compounds from daidzein and genistein, which are the typical constituent compounds of isoflavones:

[Reaction Scheme 2]

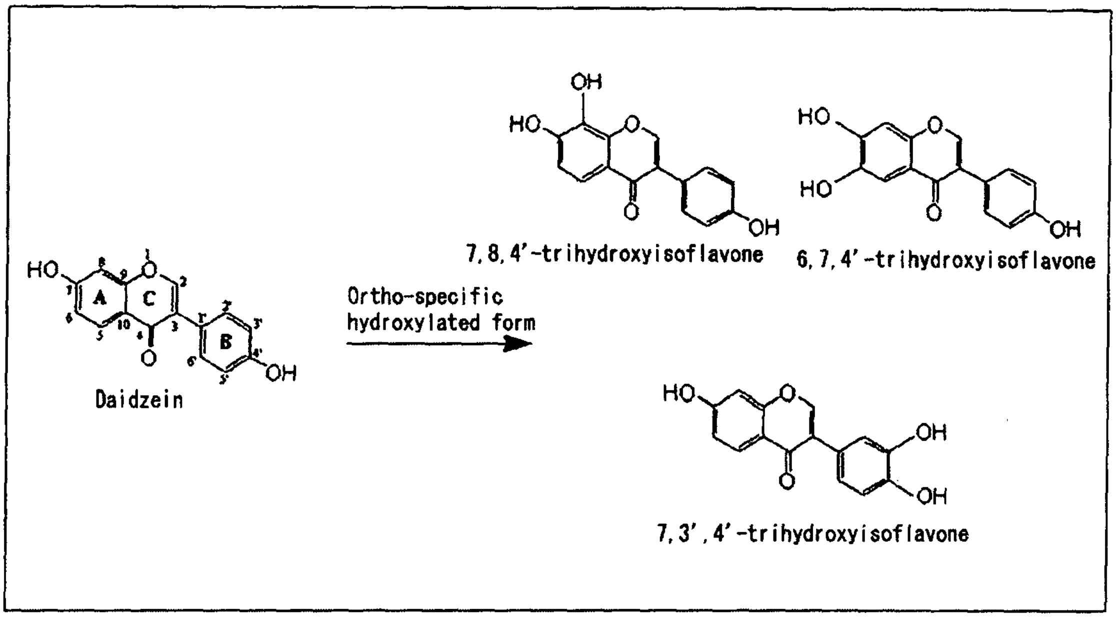


Daidzein

7,8,4'-trihydroxyisoflavone

6,7,4'-trihydroxyisoflavone

7,3',4'-trihydroxyisoflavone

Ortho-dihydroxyisoflavones produced include 7,8,4'-trihydroxyisoflavone, 7,6,4'-trihydroxyisoflavone, 7,3',4'-trihydroxyisoflavone and 7,5,3',4'-tetrahydroxyisoflavone.

Four desired position-specific forms: 7,8,4'-trihydroxyisoflavone, 7,6,4'-trihydroxyisoflavone, 7,3',4'-trihydroxyisoflavone and 7,5,3',4'-tetrahydroxyisoflavone (Orobol), could be produced using microbial strains having high substrate-specific reactivity (in the case like Orobol, biosynthesis using microorganisms is the first in the world).

Ortho-dihydroxyisoflavones could be produced using a modified reactor and reaction time.

Reaction conditions according to the present invention are as follows: For smooth oxygen supply to a microbial strain during a reaction, coils or glass beads are used in a batch reactor. A microbial strain primarily cultured in a test tube is subcultured in a 1-liter conical flask for 48 hours, and then the reactivity thereof is examined using a fresh medium. Herein, the medium is preferably ISP2, YEME or R2YE. Among them, R2YE is preferably in view of the growth reactivity of the strain. The reaction time is examined at an interval of 12 hours. When the reaction was carried out for 24 hours, the production of 7,3',4'-trihydroxyisoflavone and 7,5,3',4'-tetrahydroxyisoflavone (Orobol) was increased, and after 36 hours of the reaction, the production of 7,8,4'-trihydroxyisoflavone and 7,6,4'-trihydroxyisoflavone was increased, and after 48 hours of the reaction, modified isoflavones could be obtained (FIG. 8). The concentration (per reaction volume) of substrate used is 10 mM, and the culture of the strain and the reaction are carried out 28 °C.

The ortho-dihydroxyisoflavones produced are polar compounds having antioxidant or whitening effects.

The amount of ortho-dihydroxyisoflavones and modified isoflavones, which are rarely present in vegetable materials, can be greatly increased through biosynthesis using, as a raw material, daidzein or genistein which are the constituent components of most isoflavones.

Daidzein and genistein, used as substrates in the present invention, were purchased from Bioland Co., Ltd. (Korea).

In the present invention, *Streptomyces avermitilis* which is an actinomycete microorganism is cultured and examined for reactivity with daidzein. Based on the examination results, ortho-dihydroxyisoflavone compounds are collected and purified from the cultured material using the specific gravity difference of ethyl acetate (EA). In other words, by examining the reactivity of daidzein with actinomycete *Streptomyces avermitilis,* the production of orthodihydroxyisoflavones can be accumulated and the antioxidant ortho-dihydroxyisoflavones can be prepared from the cultured material.

[Mode for Invention]

Hereinafter, the present invention will be described in further detail with reference to examples. It is to be understood, however, that these examples are for illustrative purposes and are not to be construed to limit the scope of the present invention.

<u>Example 1: Examination of reactivity of daidzein and genistein from gram-positive *Streptomyces Avermitilis*</u>

Microorganisms performing a hydroxylation reaction of attaching an -OH group specifically at the 6, 8 or 3' position of daidzein were screened and the enzymes thereof were examined.

First, substrate specificities for daidzein and genistein were examined with yeasts, fungi and bacteria, which had different compositions. Among the examined microbial strains, *Streptomyces Avermitilis, Nocardia farcinica)* and *Streptomyces lincolnesis* showed reactivity with daidzein. Among them, *Streptomyces Avermitilis* showed the highest substrate specificity for daidzein and genistein.

As the reaction medium, an ISP2 medium was used, and a batch reactor shown in FIG. 4 was used instead of Eppendorf in the reaction. After 24 hours of the reaction, the reaction products were extracted with ethyl acetate, evaporated using a vacuum centrifuge, and then analyzed. The structures of the reaction products were analyzed using HPLC and NMR analysis systems, and the analysis results are shown in FIGS. 1 to 3. The final concentration of the substrate in the reaction was 10 mM. The results of 1H NMR spectrum analysis of the reaction products are as follows:

7,3',4'-trihydroxyisoflavone: $\delta$ 8.37 (s, H-2), 7.97 (d, J=8.5 Hz, H-5), 7.56 (d,d, J=8.0 and 2.5 Hz, H-6'), 7.53 (d, J=2.5 Hz, H-2'), 7.42 (d, J=8.0 Hz, H-5'), 6.95 (d,d, J=8.5 and 2.5 Hz, H-6), and 6.87 (d, J=2.5 Hz, H-8).
7,5,3',4'-tetrahydroxyisoflavone: $\delta$ 7.97 (s, H-2), $\delta$ 6.22 (d, J=2 Hz, H-6), 6.95 (d, J=2Hz, H-2'), 6.86 (d, J=8Hz, H-5'), 6.91 (d,d, J=2 and 8Hz, H-6').

Example 2: Examination of changed reactivity of daidzein with gram-positive *Streptomyces Avermitilis*

Not only hydroxylation reactivity at the 3' position, but also hydroxylation reactivity at the 6 and 8 positions were examined using *Streptomyces Avermitilis.* Coils were placed for smooth oxygen supply to microorganisms, and a conical flask was used to examine the reactivity (FIG. 4(B)). An R2YE medium was used for the culture of the strain, and the reaction speed was 220 rpm. After 24 hours of the reaction, the reaction products were extracted with ethyl acetate (EA), and then analyzed using GC-MS. An authentic sample could be analyzed in the GC chromatogram through different retention times. The molecular weight of each compound could be analyzed through the MS spectrum by BSTFA derivatization, and the analysis results are shown in FIG. 5 (daidzein, room temperature, 18.5 min, MS 398; 7,3',4'-trihydroxyisoflavone, room temperature, 24.4 min, MS 486; 7,8,4'-trihydroxyisoflavone, room temperature, 25.1 min, MS 486; 7,6,4'-trihydroxyisoflavone, room temperature, 26.8 min, MS 486).

Example 3: Examination of estimated reactivity of daidzein with gram-positive *Streptomyces Avermitilis*

As confirmed in Example 2, *Streptomyces Avermitilis* had reactivity with ODI. When the reaction time was one month, various modified compounds in a monohydroxylated form, a monomethoxylated form, a dihydroxylated form, a dimethoxylated form, a trihydroxylated form and a trimethoxylated form could be analyzed by GC-MS. The results of modification degree and analysis of possible reaction products are shown in FIGS. 6 to 10. It was reported that daidzein, formononetin having methylation instead of hydroxylation at the C-4 position of B-ring of daidzein, genistein and biochanin A having methylation instead of hydroxylation at the C-4 position of B-ring of genistein have not only antioxidant effects, but also antioxidant effects upon exposure to UV light (Widyarini et al. 2001).

Example 4: Measurement of melanin production inhibitory effects using Mel-Ab cells

The melanin production inhibitory effects of the ODIs obtained in Examples 1 and 2 were measured using Mel-Ab cells.

First, C57BL/6 mouse melanocyte cells (Mel-Ab cells) (Dooley, T.P. et al, Skin pharmacol, 7, pp 188-200) were cultured in a DMEM (Dulbeccos modified Eagles media) medium containing 10% fetal bovine serum, 100 nM 2-O-tetradecanoylphorbol-13-acetate and 1 nM cholera toxin in conditions of 37 °C and 5% $CO_2$. The cultured Mel-Ab cells were detached by 0.25% trypsin-EDTA and cultured in a 24-well plate at a concentration of $10^5$ cells/well. During the culture period, 100 ppm hydroquinone and each of the ODIs obtained in Examples 1 and 2 were added to the cells continuously during 3 days from 2 days after the start of the culture. Herein, the hydroquinone was used as a positive control group. The culture broth was collected and washed, and the cells were lysed with 1N sodium hydroxide and measured for absorbance at 400 nm. Based on the measurement results for absorbance, the melanin production inhibition of each compound was calculated according to the following Math Figure 1, and the calculation results are shown in Table 1 below (Dooley's method).

【Math Figure 1】

Melanin production inhibition (%)= 100-(absorbance of each test substance/absorbance of control group x 100)

[Table 1]

| Test substances | Melanin production inhibition (%) |
|---|---|
| 7,3',4'-trihydroxyisoflavone | 38.7 |
| 7,5,3',4'-tetrahydroxyisoflavone | 40.8 |
| 7,8,4'-trihydroxyisoflavone | 39.8 |
| 7,6,4'-trihydroxyisoflavone | 38.8 |
| Hydroquinone (positive control) | 41.1 |

As shown in Table 1 above, the ODIs obtained in Examples 1 and 2 of the present invention showed melanin production inhibitory effects similar to that of hydroquinone.

## Claims

**1.** A method for preparing 7,5,3',4'-tetrahydroxyiso flavone, which comprises a step of biotransforming daidzein or genistein by *Streptomyces avermitilis.*

**2.** The method of Claim 1, wherein the biotransformation is performed by position-specific hydroxylation at the 3' position of daidzein or genistein.

**3.** The method of Claim 1, wherein the biotransformation is carried out in a batch reactor into which oxygen can be supplied.

## Patentansprüche

**1.** Verfahren zum Herstellen von 7,5,3',4'-Tetrahydroxyisoflavon, welches einen Schritt der Biotransformation von Daidzein oder Genistein durch *Streptomyces avermitilis* umfasst.

**2.** Verfahren nach Anspruch 1, wobei die Biotransformation durch positionsspezifische Hydroxylierung in der 3'-Position von Daidzein oder Genistein erfolgt.

**3.** Verfahren nach Anspruch 1, wobei die Biotransformation in einem diskontinuierlichen Reaktor durchgeführt wird, in den Sauerstoff zugeführt werden kann.

## Revendications

**1.** Procédé de préparation de la 7,5,3',4-tétrahydroxyisoflavone, qui comprend une étape consistant à biotransformer la daidzéine ou la génistéine par *Streptomyces avermitilis*.

**2.** Procédé selon la revendication 1, dans lequel la biotransformation est réalisée via une hydroxylation position-spécifique au niveau de la position 3' de la daidzéine ou de la génistéine.

**3.** Procédé selon la revendication 1, dans lequel la biotransformation est réalisée dans un réacteur à fonctionnement discontinu dans lequel de l'oxygène peut être fourni.

FIG. 1

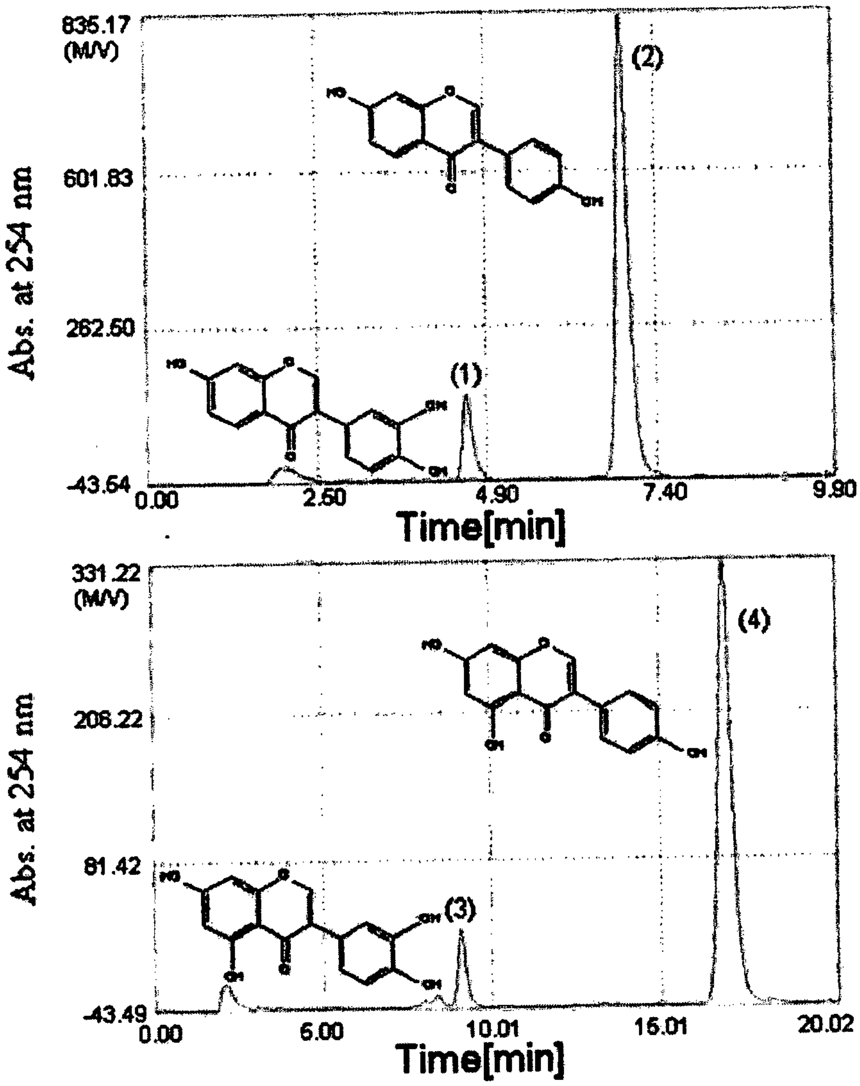
835.17
(M/V)
601.83
262.60
-43.64
Abs. at 254 nm
(1)
(2)
0.00
2.50
4.90
7.40
9.90
Time[min]
331.22
(M/V)
208.22
81.42
-43.49
Abs. at 254 nm
(3)
(4)
0.00
6.00
10.01
15.01
20.02
Time[min]

FIG. 2

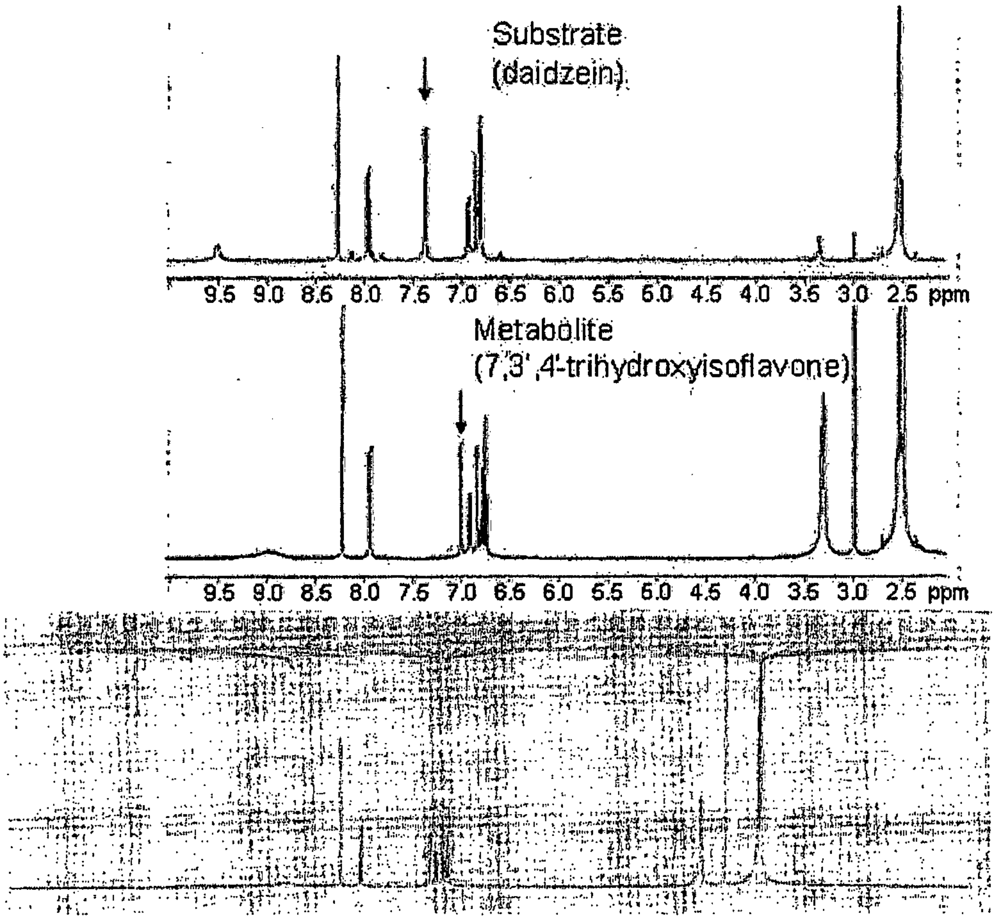
Substrate
(daidzein)
9.5 9.0 8.5 8.0 7.5 7.0 6.5 6.0 5.5 5.0 4.5 4.0 3.5 3.0 2.5 ppm
Metabolite
(7,3',4'-trihydroxyisoflavone)
9.5 9.0 8.5 8.0 7.5 7.0 6.5 6.0 5.5 5.0 4.5 4.0 3.5 3.0 2.5 ppm

FIG. 3

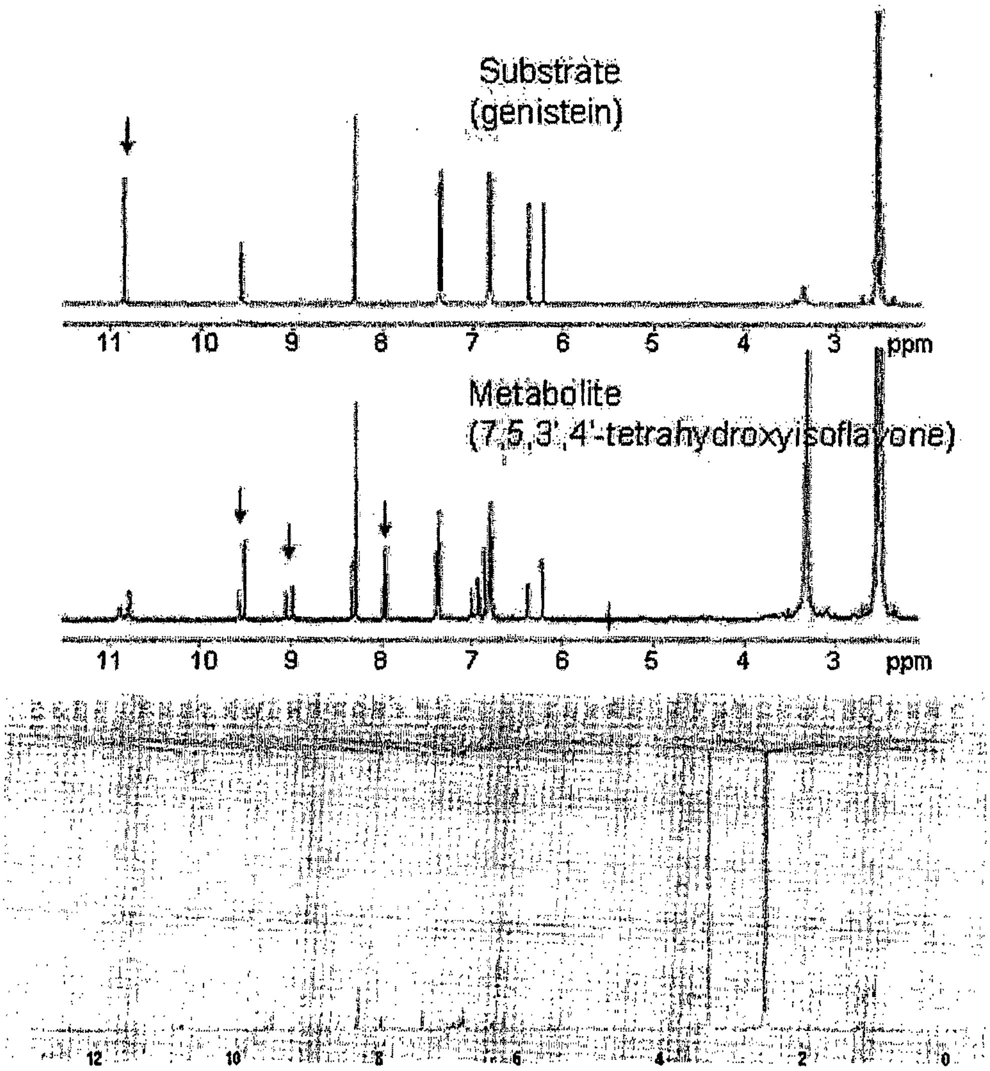
Substrate
(genistein)
11
10
9
8
7
6
5
4
3
ppm
Metabolite
(7,5,3',4'-tetrahydroxyisoflavone)
11
10
9
8
7
6
5
4
3
ppm
12
10
8
6
4
2
0

FIG. 4

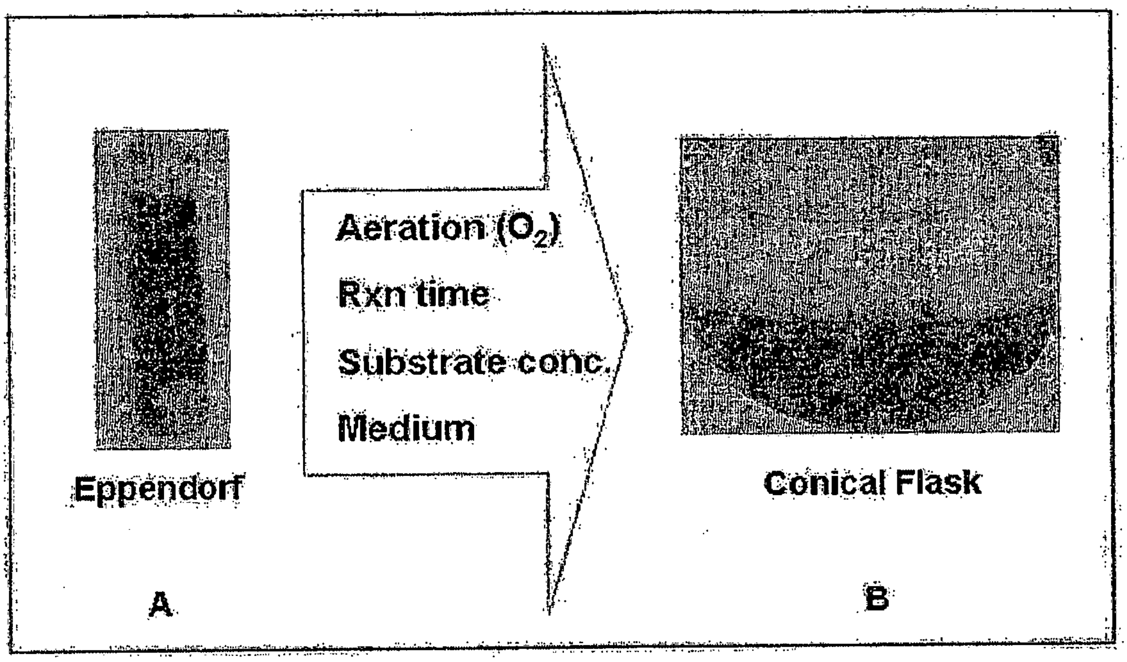
Eppendorf
Aeration ($O_2$)
Rxn time
Substrate conc.
Medium
Conical Flask
A
B

FIG. 5

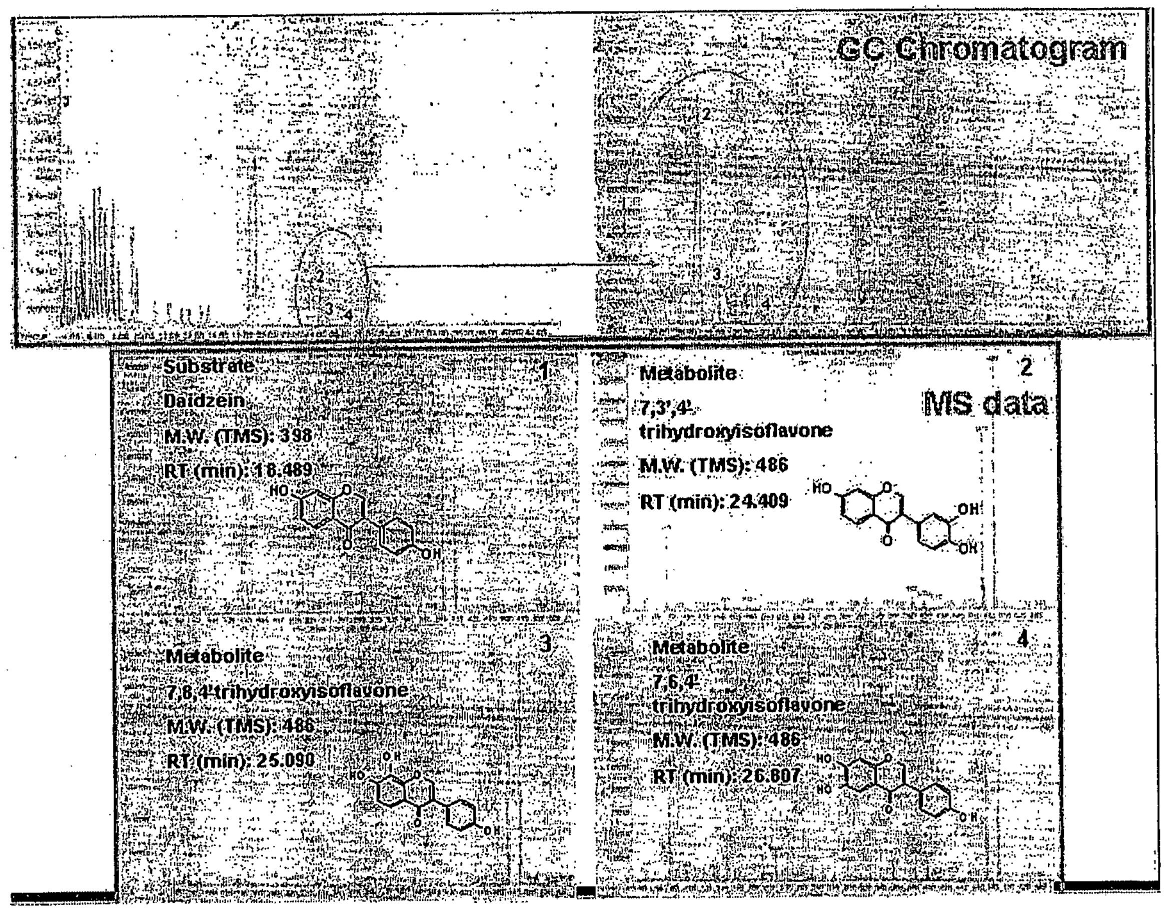
GC Chromatogram
MS data
Substrate
Daidzein
M.W. (TMS): 398
RT (min): 18.489
1
Metabolite
7,3',4'-
trihydroxyisoflavone
M.W. (TMS): 486
RT (min): 24.409
2
Metabolite
7,8,4'-trihydroxyisoflavone
M.W. (TMS): 486
RT (min): 25.090
3
Metabolite
7,6,4'-
trihydroxyisoflavone
M.W. (TMS): 486
RT (min): 26.807
4

FIG. 6

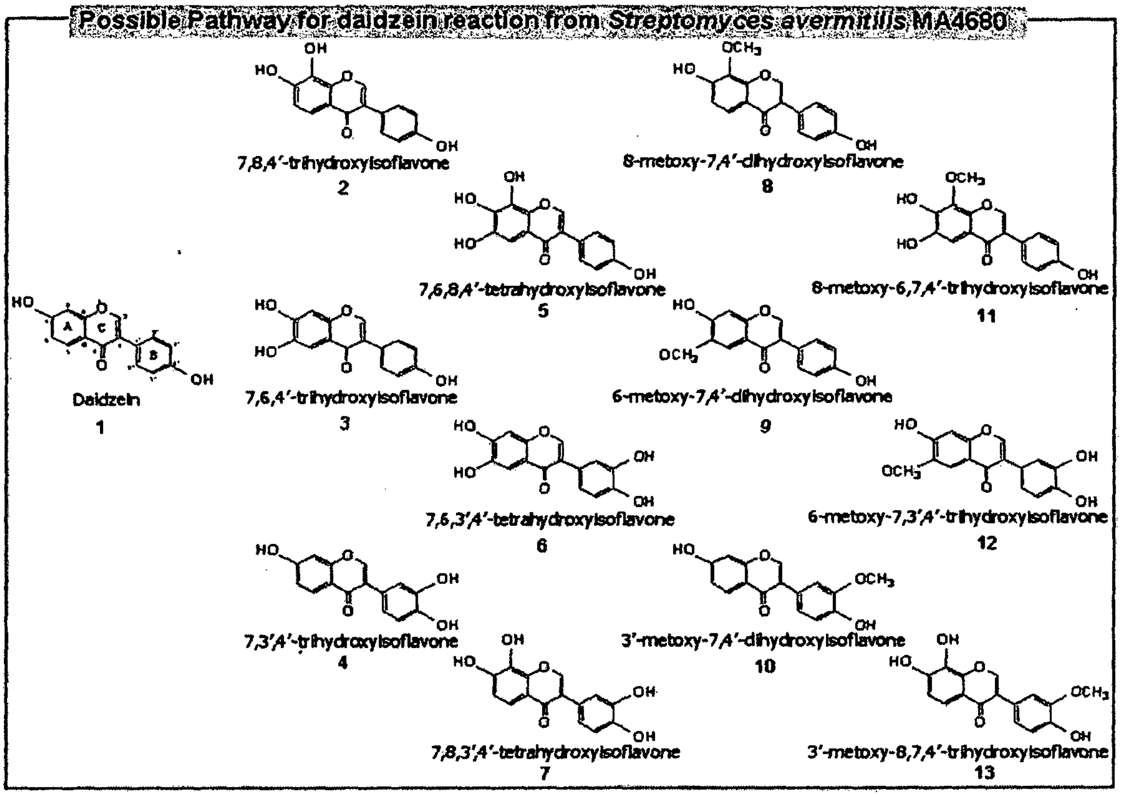

Possible Pathway for daidzein reaction from *Streptomyces avermitilis* MA4680

Daidzein
1

7,8,4'-trihydroxyisoflavone
2

7,6,4'-trihydroxyisoflavone
3

7,3',4'-trihydroxyisoflavone
4

7,6,8,4'-tetrahydroxyisoflavone
5

7,6,3',4'-tetrahydroxyisoflavone
6

7,8,3',4'-tetrahydroxyisoflavone
7

8-metoxy-7,4'-dihydroxyisoflavone
8

6-metoxy-7,4'-dihydroxyisoflavone
9

3'-metoxy-7,4'-dihydroxyisoflavone
10

8-metoxy-6,7,4'-trihydroxyisoflavone
11

6-metoxy-7,3',4'-trihydroxyisoflavone
12

3'-metoxy-8,7,4'-trihydroxyisoflavone
13

FIG. 7

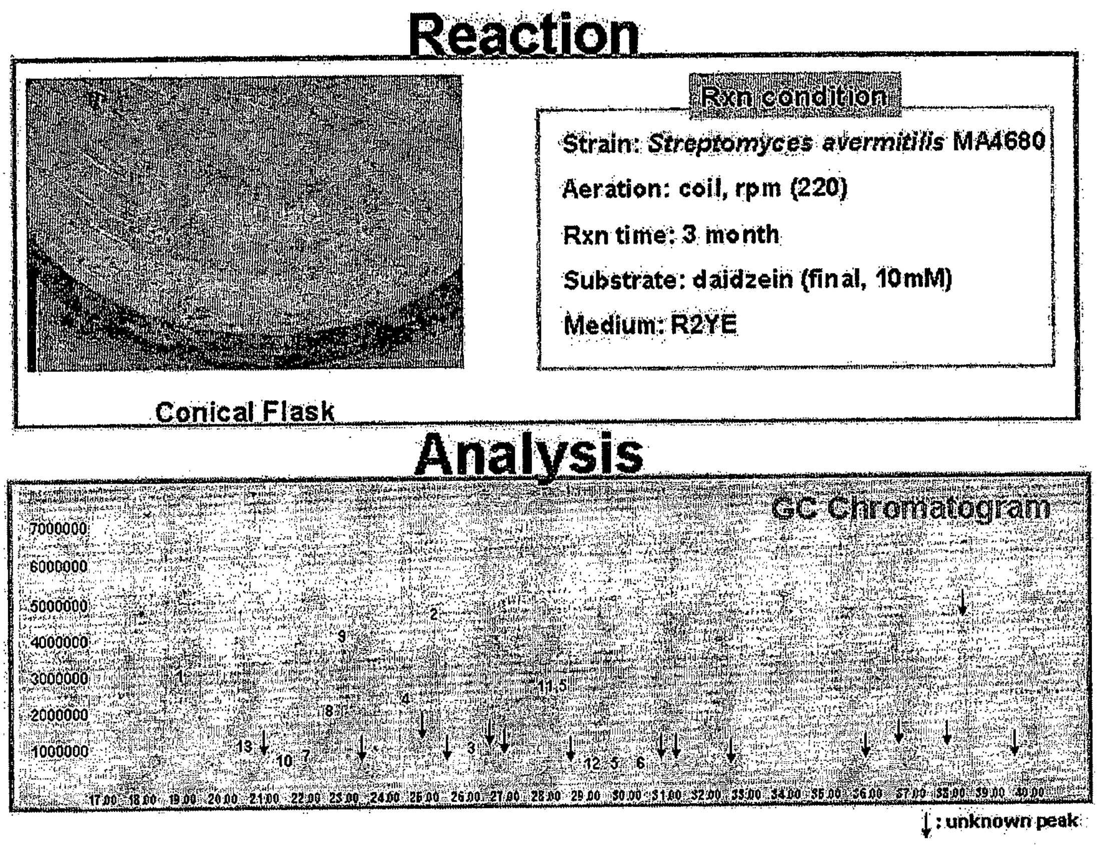
Reaction
Rxn condition
Strain: Streptomyces avermitilis MA4680
Aeration: coil, rpm (220)
Rxn time: 3 month
Substrate: daidzein (final, 10mM)
Medium: R2YE
Conical Flask
Analysis
GC Chromatogram
7000000
6000000
5000000
4000000
3000000
2000000
1000000
17.00 18.00 19.00 20.00 21.00 22.00 23.00 24.00 25.00 26.00 27.00 28.00 29.00 30.00 31.00 32.00 33.00 34.00 35.00 36.00 37.00 38.00 39.00 40.00
↓: unknown peak

FIG. 8

# GC-MS Analysis: Modified Isoflavones

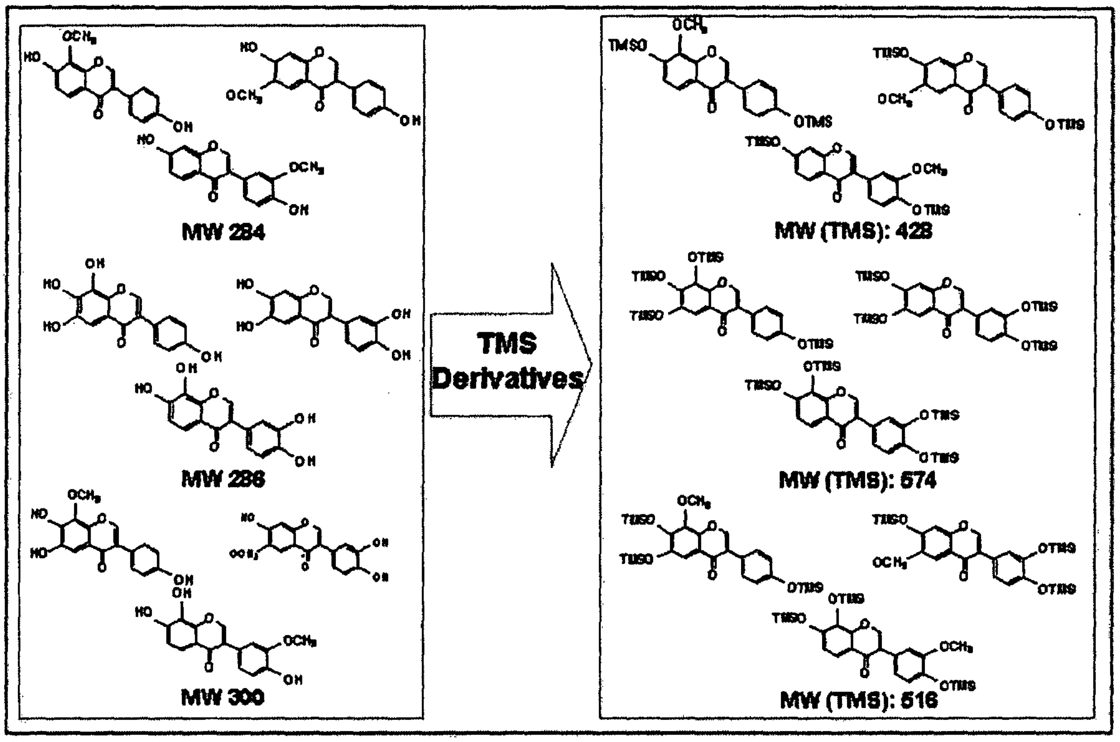

FIG. 9

MS data

Substrate
Daidzein
M.W. (TMS): 398
RT (min): 18.725

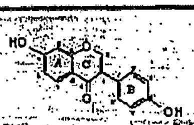

1

Metabolite
8-ODI
M.W. (TMS): 486
RT (min): 25.243

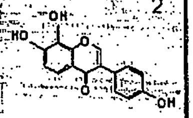

2

Metabolite
6-ODI
M.W. (TMS): 486
RT (min): 26.233

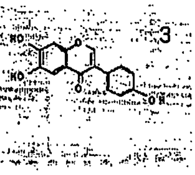

3

Metabolite
3'-ODI
M.W. (TMS): 486
RT (min): 24.564

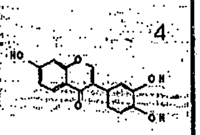

4

Metabolite
7,6,8,4'-tetrahydroxyisoflavone
M.W. (TMS): 574
RT (min): 28.959

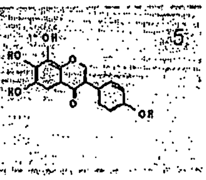

5

Metabolite
7,6,3',4'-tetrahydroxyisoflavone
M.W. (TMS): 574
RT (min): 30.906

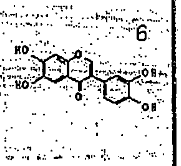

6

Metabolite
7,8,3',4'-tetrahydroxyisoflavone
M.W. (TMS): 574
RT (min): 22.177

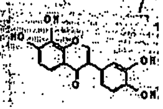

7

Metabolite
8-methoxy-7,4'-dihydroxyisoflavone
M.W. (TMS): 428
RT (min): 22.625

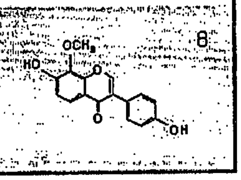

8

FIG. 10

**MS data**

Metabolite 9
6-methoxy-7,4'-dihydroxyisoflavone
M.W. (TMS): 428
RT (min): 22.991

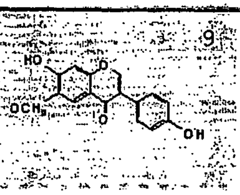

Metabolite 10
3'-methoxy-7,4'-dihydroxyisoflavone
M.W. (TMS): 428
RT (min): 21.994

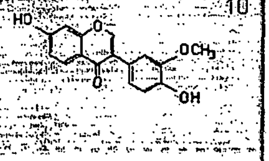

Metabolite 11
8-methoxy-6,7,4'-trihydroxyisoflavone
M.W. (TMS): 516
RT (min): 28.634

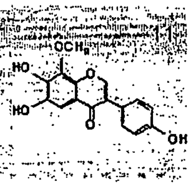

Metabolite 12
6-methoxy-7,3',4'-trihydroxyisoflavone
M.W. (TMS): 516
RT (min): 29.237

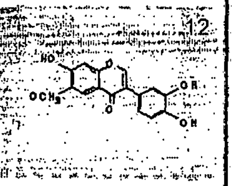

Metabolite 13
3'-methoxy-8,7,4'-trihydroxyisoflavone
M.W. (TMS): 516
RT (min): 20.502

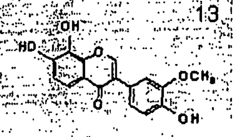

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **B. PANDEY et al.** *Bioconversion of daidzein to ortho dihydroxy isoflavone by recombinant Streptomyces avermitilis,* 2007
- **C. ROH et al.** *Isolation of novel hydroxylated isoflavones, ODI (ortho-dihydroxyisoflavone) from traditional Korean soybean paste (Doenjang) and isoflavone's biotransformation study,* 2007, http://www.earticle.net/article.aspx?sn=98606
- **K. KOMIYAMA et al.** Isolation of isoflavonoids possessing antioxidant activity from the fermentation broth of Streptomyces sp. *The Journal of Antibiotics,* 1989, vol. 42 (9), ISSN 0021-8820, 1344-1349
- **S. FUNAYAMA et al.** Structural study of isoflavonoids possessing antioxidant activity isolated from the fermentation broth of Streptomyces sp. *The Journal of Antibiotics,* 1989, vol. 42 (9), ISSN 0021-8820, 1350-1355
- **J.H. KIM.** ODI (o-dihydroxyisoflavone) biosynthesis and ODI production from Doenjang (Fermented Soybean paste) and Streptomyces avermitilis. *Spring symposium of the Korean Society of Industrial and Engineering Chemistry,* 11 May 2007
- **DOOLEY, T.P. et al.** *Skin pharmacol,* vol. 7, 188-200